# EUROPEAN PATENT APPLICATION

(11) **EP 1 704 867 A1**
(43) Date of publication of application: **27.09.2006**
(21) Application number: 05075656.8
(22) Date of filing: 18.03.2005
(51) Int. Cl.: A61K 38/55, A61K 38/48, A61K 39/40, A61K 39/02, C12Q 1/56, C12Q 1/37

(54) **Cross-beta structures on microbial organisms**

(71) Applicant: Crossbeta Biosciences B.V., 3584 CX Utrecht (NL)
(72) Inventor: Gebbink, Martijn Frans Ben Gerard, 3755 HL Eemnes (NL); Bouma, Barend, 3994 CE Houten (NL); Wösten, Herman Abel Bernard, 3705 SN Zeist (NL)
(74) Representative: Winckels, Johannes Hubertus F.

(57) **Abstract**

The present invention discloses the use of a protease inhibitor in the preparation of a medicament for the treatment of microbial infections. The invention further discloses the use of a compound binding to a cross-ß structure or an antibody specific for a cross-ß structure in the preparation of a medicament for the treatment of microbial infections.

The invention further discloses methods for attenuating microbial pathogens by deleting at least part of a gene encoding a cross-ß structure forming protein. The invention also discloses antimicrobial composition, and a kit for detecting microbial contamination in a solution or a substance.

## Description

The invention relates to the field of microbiology, more specifically to antimicrobial medicines and antimicrobial immunogenic compositions.

Amyloid fibrils have been associated with pathology in a class of degenerated diseases like for example Alzheimer's disease and Creutzfeldt-Jakob. Amyloid structures also occur on the surface of microbial organisms like fungi and bacteria. The proteins in amyloid fibrils in degenerative diseases like Alzheimer's disease and Creutzfeldt-Jakob differ from those on the surface of bacteria and fungi.

Said amyloid-like structures are generally called hydrophobins on fungi, chaplins on gram-positive bacteria, and curli or tafi or aggregative fimbriae on gram-negative bacteria.

Generally the virulence of a micro-organism depends on characteristics like for example colonization factors, exo-and endotoxine production, replication time, resistance to antibiotics, and survival in the host macrophages, just to name a few. Methods to attenuate microorganisms for vaccination purposes were generally directed to the above-mentioned characteristics. Antimicrobial medicines are generally antibiotic compounds or bacteriostatic compounds

Since resistance of microorganisms to antibiotic and bacteriostatic compounds is an ever-increasing problem, new methods for combating microorganisms are needed.

The present invention discloses that microbial amyloid-like structures contain a cross- β structure. Microbial hydrophobins, chaplins, tafi's, curli's and aggregative fimbriae contain a cross- β structure and are all capable of inducing protease activity in the body of a host. By inducing protease activity in a host tissue, a microorganism comprising a cross- β structure indirectly weakens the intercellular matrix thereby decreasing the integrity of the tissue. By this mechanism, said microorganism is able to invade the body.

Furthermore, according to the invention said microbial cross- β structure is capable of binding intravascularly to factor XII. Binding of factor XII to said cross- β structure is followed by conversion of factor XII into a serine protease factor XIIa. Said factor XIIa is involved in the release of bradykinine, which induces amongst other things, hemostasis and the release of more tissue type plasminogen activator (tPA) which is also a serine protease. Hence microorganisms comprising a cross- β structure use host cell's proteases to weaken the intracellular matrix of tissue and to cause intravascular hemostasis. Therefore, the presence of amyloid fibrils comprising a cross- β structure renders a microorganism more virulent for a host.

The identification in the present invention of a cross- β structure in said surface proteins of microorganisms now provides new methods for decreasing the virulence of a micro-organism and offers new methods for inhibiting infection of a host by said microorganism.

The present invention shows that a protease inhibitor is capable of decreasing the serine protease activity caused by a cross- β structure. Therefore, the dissolving action on tissue and on blood clots is decreased, and the invasion of tissue by the microbial organism is at least decreased. Administration of said protease inhibitor as a medicine is very useful for treatment of an animal and/or a human being suffering from an infection with a microbial organism. Therefore, the present invention discloses the use of a protease inhibitor in the preparation of a medicament for the treatment of microbial infections.

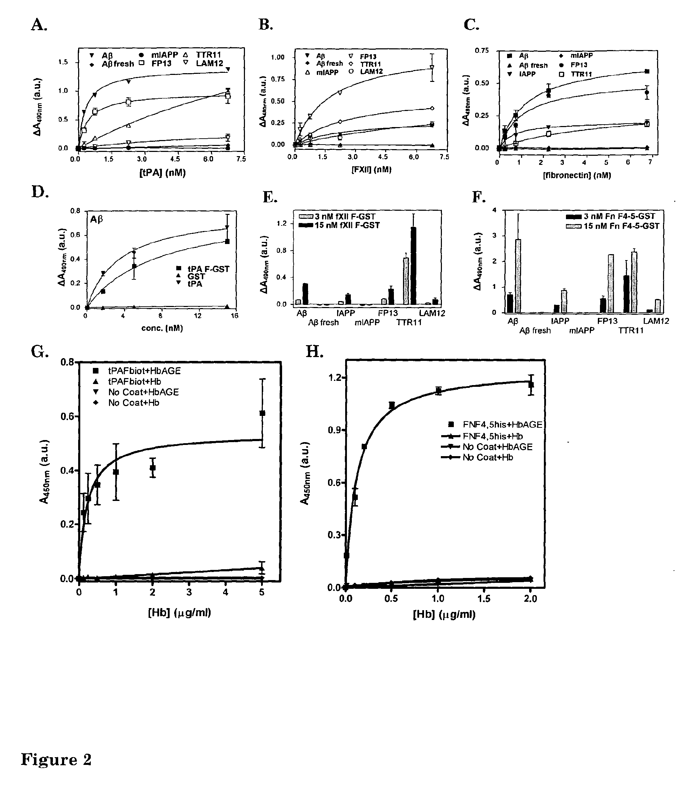

Of course, a compound binding to a cross-β structure also prevents the increase in protease activity and is therefore a potent inhibitor of a microbial infection. Therefore, the present invention also discloses the use of a cross-β structure binding compound in the preparation of a medicament for the treatment of microbial infections. Preferably, said compound comprises an antibody, or a functional fragment or derivative thereof, and/or a finger domain of a protease, specific for a cross-β structure. An example of such a finger domain is described in example 2 and in figure 2. Most preferably said antibody comprises a bi-specific antibody capable of recognizing and binding to a cross-β structure.

Modern technology has enabled a skilled person to produce and select monoclonal antibodies. In this development also bi-specific antibodies are constructed. Generally a bi-specific antibody comprises two different binding specificities in single molecule. In for example a bi-specific antibody against a cross-β structure of a microorganism, one antibody specificity is directed against said cross- β structure, while the other antibody specificity is preferably directed against another antigen of said micro-organism. A bi-specific antibody has the advantage that the binding rate is increased because the antibody binds to more than one antigen. In one embodiment a bi-specific antibody is generated which is directed against a particular (pathogenic) strain of microorganisms while leaving other (non-pathogenic) strains of the same micro-organism untouched. Therefore, the present invention discloses the use according to the invention, wherein said antibody is a bi-specific antibody against a cross- β structure and a microbial antigen.

The above-described use of a protease inhibitor and/or antibody and/or bi-specific antibody is useful for the treatment of infections with microorganisms. Both gram ―negative and gram-positive bacteria and also fungi have a cross- β structure on the surface. Therefore, the present invention discloses a use according to the invention, wherein said microorganism is a cross- β structure-positive microorganism. Because a cross- β structure on a gram-positive microorganism is generally present in a specific structure like a hydrophobin, or chaplin, the present invention discloses the use according to the invention, wherein a cross-β structure comprises a hydrophobin or a chaplin.

Because a hydrophobin generally occurs on a fungus, the present invention discloses the use according to the invention, wherein said microorganism is a fungus. Hydrophobins have been detected in both the ASCOMYCETES and the BASIDIOMYCETES. Both phyla represent most species in the fungal kingdom. Hydrophobins are generally expressed in dimorphic fungi. For the present invention, treatment of infections with pathogenic fungi are of preferred interest.

Because a chaplin generally occurs on a gram-positive bacterium, the present invention discloses a use according to the invention, wherein said microorganism is a gram-positive bacterium. In gram-positive bacteria, chaplins are generally found on *Streptomyces* species and in other actinomycetes. Therefore, the invention provides a use, wherein said gram-positive bacterium is an actinomycete or a streptomycete bacterium.

Because a curli or a Tafi or a thin aggregative fimbria generally occurs on gram negative bacteria, the present invention discloses a use according to the invention, wherein said microorganism is a gram negative bacterium.

Important pathogens covered with curli or tafi or thin aggregative fimbriae are bacteria from the genera E.coli and Salmonella. Therefore, the present invention discloses a use according to the invention wherein said gram-negative bacterium is an *E.coli* bacterium or a *Salmonella* bacterium.

By disclosing the function of a cross- β structure in the amyloid fibril for the virulence of a microorganism, the present invention, discloses a method for decreasing the virulence of a microorganism by at least in part preventing the formation of cross- β structures. Said decreasing is preferably achieved by deleting at least a part of a gene encoding an amyloid fibril forming protein. This results in at least partly decreased amyloid fibre formation on the surface, and therefore decreased cross- β structure presence. Said microorganism with decreased virulence is safer for a host and is useful as an immunogenic composition. Therefore, the present invention discloses a method for producing a less virulent microorganism, comprising deleting at least a part of a gene of said microorganism encoding an amyloid fibril forming protein.

Because part of the amyloid fibril is formed by a cross-β structure, decreasing the amount of a cross-β structure-forming protein is a suitable way of decreasing the virulence of a microorganism without deleting other antigens. Such a microorganism preferably is more suitable for use in an immunogenic composition than a microorganism from which antigens have been stripped or altered in a process of attenuation. Therefore, the present invention discloses an immunogenic composition comprising a microorganism from which at least one gene, encoding a cross-β structure forming protein, has been at least in part deleted.

In a preferred embodiment of the invention a protease inhibitor of the invention is used in combination with a fungicide compound. Said fungicide preferably potentiates the effect of said protease inhibitor in decreasing an infection with a pathogenic fungus. A fungicide preferably is a compound generally known for its fungicidal activity. In this invention, for application to a host, (such as an animal or a human being), use of fungicidal compounds is restricted to those fungicides that are suitable for use on or in said host. Therefore, the present invention discloses a composition comprising a protease inhibitor and a fungicide.

In another preferred embodiment, a protease inhibitor of the invention is used in combination with a bactericide compound. Said bactericide preferably potentiates the effect of said protease inhibitor in decreasing an infection with a pathogenic bacterium. In this invention, for application to a host (such as an animal or a human being), use of bactericidal compounds is restricted to those bactericides that are permitted for use on or in said host. Therefore, in a preferred embodiment, the present invention discloses a composition comprising a protease inhibitor and a bactericide.

Because a cross-β structure induces serine protease enzymes, inhibition of said serine protease enzymes by specific serine protease inhibitors is preferred. Therefore, the present invention discloses a composition according to the invention, wherein said protease inhibitor is a serine protease inhibitor.

The invention further provides a composition comprising a protease inhibitor and a cross-β structure-binding compound. Such composition is particularly suitable for counteracting the microbial cross-β structure activity. Said cross-β structure-binding compound preferably comprises an antibody, or a functional fragment or derivative thereof, and or a finger domain of a protease, specific for a cross-β structure. In order to specifically counteract bacteria and/or fungi said composition preferably further comprises a bactericide and/or a fungicide.

In another embodiment, the invention provides a kit for detecting microbial contamination of a solution and/or a substance, said kit comprising a cross-β structure binding compound and a means for detecting binding of said cross-β structure to said binding compound. Said means for detecting binding of a cross-β structure to a cross-β structure-binding compound preferably comprises a tPA- and/or factor XII activation assay. In another preferred embodiment said means for detecting binding of a cross-β structure to a cross-β structure binding compound comprises visualization of a staining compound.

The invention is further explained in the following examples, without being restricted to them.

### Example 1

### Bacterial cells with amyloid-like core protein activate the fibrinolytic system in vitro

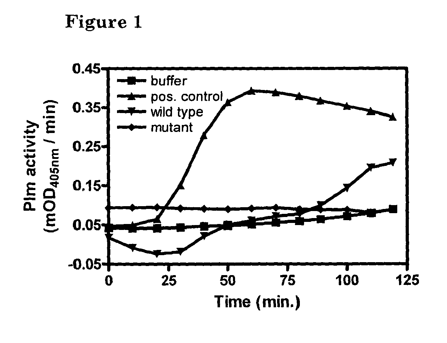

The Streptomyces coelicolor bacterium strain comprises a family of core proteins, chaplins A-H, which have adopted amyloid-like fibril conformation(Claessen, Rink et al., 2003a). We now show that contacting the wild type strain with tPA, plasminogen and plasmin substrate S-2251 results in activation of tPA and plasminogen (Fig. 1). Interestingly, a mutant strain that lacks the amyloid-like core protein, does not stimulate tPA activation (Fig. 1).

The data show that the presence of the chaplin core proteins with amyloid-like conformation, on the surface of Streptomyces coelicolor cells activates the fibrinolytic pathway, by activation of tPA. Cells of a mutant strain lacking the genes that encode for the amyloid-like chaplin do not induce tPA activation. Activation of the fibrinolytic cascade or of factor XII, the key protein in the contact system of blood coagulation, by several different pathogens shows that in general core proteins are involved in interactions with the host haemostatic system. Our determination now of the F domain as the specific domain that interacts with protein aggregates comprising amyloid-like cross-β structure conformation enables for tracing of pathogens with exposed amyloid-like proteins on their surface. The tPA- and factor XII activation assays are indicative in quality measures of solutions suspected for the presence of pathogenic infections.

### Example 2

### tPA, factor XII, fibronectin and the fibronectin type I domains of tPA, factor XII and fibronectin bind to protein aggregates with cross-β structure conformation

Previously, we established that tissue-type plasminogen activator specifically interacts with protein and peptide aggregates that comprise a cross-β structure conformation, a structural element found in amyloid-like polypeptide assemblies(Kranenburg, Bouma et al., 2002; Bouma, Kroon-Batenburg et al., 2003). Now, we expanded this analysis to other proteins that resemble tPA domain architecture and we separated domains of tPA. Binding of full-length tPA, factor XII and fibronectin, as well as of fibronectin type I (finger, F) domains of tPA and factor XII and F4-5 of fibronectin, to protein and peptide aggregates with cross-β structure conformation was analyzed in an ELISA. In Fig. 1 it is shown that the full-length proteins as well as the recombinant F domains bind specifically to cross-β structure rich compounds. Binding of tPA and factor XII was established for immobilized amyloid-β(1-40) (Aβ) with amyloid-like properties, fibrin peptide FP13, that encompasses the tPA activating sequence 148KRLEVDIDIKIR160 of the fibrin α-chain, TTR11, which is an 11 amino-acid residues peptide from transthyretin that forms cross-β structure, and LAM12, which is a 12 amino-acid residues peptide from laminin that forms cross-β structure (Fig. 1A, B) Negative controls were freshly dissolved, monomerized Aβ and non-amyloid murine islet amyloid polypeptide (mIAPP). For fibronectin, human amyloid IAPP is depicted instead of LAM12 (Fig. 1C). The separate F domains also bind to aggregates with cross-β structure, as depicted for Aβ and tPA F in Fig. 1D, and for all aggregates and factor XII F and fibronectin F4-5 in Fig. 1E and F. In addition, immobilized tPA F with a biotin tag and fibronectin F4-5 with a His-tag specifically capture glycated haemoglobin with amyloid-like properties in solution (Fig. 1G, H).

### Material and Methods

### Cloning and expression of recombinant fibronectin type I domains

Amino-acid sequences of recombinantly produced domains of tPA, fibronectin and factor XII, and the domain architecture of the recombinant constructs are depicted in Box I. Amino-acid residue numbering is according to SwissProt entries. Each construct has a carboxy terminal GST-tag (GST). Factor XII fibronectin type I domain (F) and fibronectin F4-5 are preceded by two amino acids (GA), following the C-terminus of the tPA propeptide. All F constructs are followed by the (G)RP sequence derived from the original pMT2-GST vector. For each recombinant construct, the oligonucleotides that were used for PCR are listed in Box I. The relevant restriction sites are underlined. The tPA fibronectin type I domain (F, finger domain), together with the tPA propeptide, was amplified using 1 ng vector Zpl7 containing tPA(Johannessen, Diness et al., 1990) and oligonucleotides 1 and 2, digested with SalI and NotI and cloned into pMT2SM-GST(Gebbink, Zondag et al., 1995). As a result *Schistosoma japonicum* glutathion-S-transferase (GST) is fused to the C-terminus of the expressed constructs. The constructs were subsequently ligated with SalI and EcoRI in pGEM3Zf(-) (Promega, Madison, WI, USA). The resulting plasmid was used as a cloning cassette for preparation of factor XII F and fibronectin F4-5 constructs. The selection of fibronectin type I domains of fibronectin was based on the following reasoning. tPA binds to fibrin with its fibronectin type I domain(van Zonneveld, Veerman et al., 1986) and competes with fibronectin for fibrin binding(Beckmann, Geiger et al., 1991). A fibrin binding-site of fibronectin is enclosed in its fibronectin type I 4-5 (Rostagno, Schwarzbauer et al., 1999). We show here that the fibronectin type I domain of tPA mediates binding to amyloid. This suggests that also the fibrin-binding fibronectin type I domains of fibronectin can bind to amyloid. All domains were cloned after the tPA propeptide using a BglII restriction site that is present between the tPA propeptide region and the F domain(Johannessen, Diness et al., 1990), and the NotI or KpnI site that is present in front of the thrombin cleavage site(Gebbink, Zondag et al., 1995). Subsequently, constructs were ligated HindIII and EcoRI in the pcDNA3.1 expression vector (Invitrogen, The Netherlands). This results in e.g. pcDNA3.1-factor XII F-GST and pcDNA3.1-Fn F4-5-GST. In addition, the GST tag alone, preceded by the tPA propeptide, was cloned into pcDNA3.1. The separate GST-tag has five additional residues at the N-terminus (GARRP). tPA cDNA was a kind gift of M. Johannessen (NOVO Research Institute, Bagsvaerd, Denmark). The cDNA encoding for factor XII was a kind gift of F. Citarella (University of Rome "La Sapienza", Italy). S.A. Newman (New York Medical College, Valhalla, USA) kindly provided the cDNA encoding for an N-terminal fragment of human fibronectin, comprising fibronectin type I domains 4-5.
Alternatively, recombinant finger domains of fibronectin (F4-5) and tPA were expressed with a His-tag. Two fibronectin F4-5 constructs were cloned. One construct comprising the Ig_{κ} signal sequence (vector 71, ABC-expression facility, Utrecht University/UMC Utrecht). With two designed primers (8, 9, see Box I) the fibronectin fragment was obtained from the construct pcDNA3.1-Fn F4-5-GST and BamHI and NotI restriction sites were introduced at the termini. In addition, cDNA encoding for a C-terminal His-tag was included in the designed primer. The cDNA fragment was cloned BglII-NotI in vector 71 that was digested with BamHI-NotI. Vector 71 has a BamHI site next to the Ig_{κ} signal sequence. See Box I for the construct details. A construct comprising the signal sequence of human growth hormone, the cDNA encoding for growth hormone (GH), an octa-His tag, a TEV cleavage site, the tPA F insert and a C-terminal hexa-His tag was made using vector 122b (ABC-expression facility). The tPA F-His cDNA was obtained using pcDNA3.1-tPA-F-GST as a template for a PCR with primers 10 and 11 (Box I). The PCR insert was digested BglII-NotI, the vector was digested BamHI-NotI. The BamHI site is located next to the GH-His-TEV sequence. A second Fn F4-5 construct was made similarly to the GH-His-tPA F-His construct

### Brief description of the drawings

**Figure 1. Streptomyces coelicolor bacteria activate the fibrinolytic system in vitro, whereas bacterium cells of the Δrdlab mutant strain, which lacks the amyloid-like core chaplins, does not.**
   Cells of the bacterium Streptomyces coelicolor wild type strain (wild type) are able to activate the tPA-mediated conversion of zymogen plasminogen to the active serine protease plasmin, as determined in a chromogenic assay with the plasmin substrate S-2251. In contrast, cells of a mutant strain Δrdlab, which lack the gene encoding for the chaplins core proteins (mutant), that has amyloid-like conformation, do not activate plasmin generation. Positive control was 100 µg/ml amyloid γ-globulins (pos. control), negative control was buffer.
**Figure 2. Binding of tPA, factor XII, fibronectin and finger domains thereof to compounds with cross-β structure.**
   **A-C.** Full-length purified tPA **(A),** factor XII **(B)** and fibronectin **(C)** bind to immobilized peptides with cross-β structure conformation, in an ELISA. **D-F.** In an ELISA, the recombinant Fibronectin type I, or finger domains (F) of tPA **(D),** factor XII **(E)** and fibronectin **(F)** bind specifically to immobilized amyloid-like peptides with cross-β structure conformation. The control free GST tag does not bind. **G.** In an ELISA, immobilized chemically synthesized fibronectin type I domain of tPA (tPAFbiotin) specifically captures glycated haemoglobin (Hb-AGE) from solution, and not control haemoglobin. **H.** In an ELISA, recombinantly expressed fibronectin type I domains 4-5 of fibronectin, N-terminally tagged to growth hormone and a His₆-tag, and C-terminally tagged to a His₆-tag (FnF4,5his), specifically captures Hb-AGE from solution, and not control haemoglobin.

### References

Bamberger,M.E., Harris,M.E., McDonald,D.R., Husemann,J., Landreth,G.E., 2003. A cell surface receptor complex for fibrillar beta-amyloid mediates microglial activation. J. Neurosci., 23, 2665-2674.

Beckmann,R., Geiger,M., de Vries,C., Pannekoek,H., Binder,B.R., 1991. Fibronectin decreases the stimulatory effect of fibrin and fibrinogen fragment FCB-2 on plasmin formation by tissue plasminogen activator. J. Biol. Chem., 266, 2227-2232.

Bellotti,V., Mangione,P., Merlini,G., 2000. Review: immunoglobulin light chain amyloidosis--the archetype of structural and pathogenic variability. J. Struct. Biol., 130, 280-289.

Bouma,B., Kroon-Batenburg,L.M., Wu,Y.P., Brunjes,B., Posthuma,G., Kranenburg,O., de Groot,P.G., Voest,E.E., Gebbink,M.F., 2003. Glycation induces formation of amyloid cross-beta structure in albumin. J. Biol. Chem., 278, 41810-41819.

Buxbaum,J.N., 2004. The systemic amyloidoses. Curr. Opin. Rheumatol., 16, 67-75.

Claessen,D., Rink,R., de Jong,W., Siebring,J., de Vreugd,P., Boersma,F.G., Dijkhuizen,L., Wosten,H.A., 2003a. A novel class of secreted hydrophobic proteins is involved in aerial hyphae formation in Streptomyces coelicolor by forming amyloid-like fibrils. Genes Dev., 17, 1714-1726.

Claessen,D., Rink,R., de Jong,W., Siebring,J., de Vreugd,P., Boersma,F.G., Dijkhuizen,L., Wosten,H.A., 2003b. A novel class of secreted hydrophobic proteins is involved in aerial hyphae formation in Streptomyces coelicolor by forming amyloid-like fibrils. Genes Dev., 17, 1714-1726.

Connor,P., Hunt,B.J., 2003. Cerebral haemostasis and antiphospholipid antibodies. Lupus, 12, 929-934.

Coraci,I.S., Husemann,J., Berman,J.W., Hulette,C., Dufour,J.H., Campanella,G.K., Luster,A.D., Silverstein,S.C., El Khoury,J.B., 2002. CD36, a class B scavenger receptor, is expressed on microglia in Alzheimer's disease brains and can mediate production of reactive oxygen species in response to beta-amyloid fibrils. Am. J. Pathol., 160, 101-112.

de Groot,P.G., Derksen,R.H., 2004. Antiphospholipid antibodies: update on detection, pathophysiology, and treatment. Curr. Opin. Hematol., 11, 165-169.

de Groot,P.G., Horbach,D.A., Derksen,R.H., 1996. Protein C and other cofactors involved in the binding of antiphospholipid antibodies: relation to the pathogenesis of thrombosis. Lupus, 5, 488-493.

de Laat,B., Derksen,R.H., Urbanus,R.T., de Groot,P.G., 2004a. IgG antibodies that recognize epitope Gly40-Arg43 in domain I of {beta}2-glycoprotein I cause LAC and their presence correlates strongly with thrombosis. Blood, ..

de Laat,H.B., Derksen,R.H., Urbanus,R.T., Roest,M., de Groot,P.G., 2004b. beta2-glycoprotein I-dependent lupus anticoagulant highly correlates with thrombosis in the antiphospholipid syndrome. Blood, 104, 3598-3602.

Gamba,G., Montani,N., Anesi,E., Palladini,G., Capezzera,M., Soldavini,E., Merlini,G., 2000. Clotting alterations in primary systemic amyloidosis. Haematologica, 85, 289-292.

Gebbink,M.F., Zondag,G.C., Koningstein,G.M., Feiken,E., Wubbolts,R.W., Moolenaar,W.H., 1995. Cell surface expression of receptor protein tyrosine phosphatase RPTP mu is regulated by cell-cell contact. J. Cell Biol., 131, 251-260.

Hackeng,T.M., Dawson,P.E., Kent,S.B., Griffin,J.H., 1998. Chemical synthesis of human protein S thrombin-sensitive module and first epidermal growth factor module. Biopolymers, 46, 53-63.

Hackeng,T.M., Fernandez,J.A., Dawson,P.E., Kent,S.B., Griffin,J.H., 2000. Chemical synthesis and spontaneous folding of a multidomain protein: anticoagulant microprotein S. Proc. Natl. Acad. Sci. U. S. A, %19;97, 14074-14078.

Hackeng,T.M., Griffin,J.H., Dawson,P.E., 1999. Protein synthesis by native chemical ligation: expanded scope by using straightforward methodology. Proc. Natl. Acad. Sci. U. S. A, 96, 10068-10073.

Hackeng,T.M., Mounier,C.M., Bon,C., Dawson,P.E., Griffin,J.H., Kent,S.B., 1997. Total chemical synthesis of enzymatically active human type II secretory phospholipase A2. Proc. Natl. Acad. Sci. U. S. A, 94, 7845-7850.

Hackeng,T.M., Rosing,J., Spronk,H.M., Vermeer,C., 2001. Total chemical synthesis of human matrix Gla protein. Protein Sci., 10, 864-870.

Hoppener,J.W., Ahren,B., Lips,C.J., 2000. Islet amyloid and type 2 diabetes mellitus. N. Engl. J. Med., 343, 411-419.

Hoppener,J.W., Oosterwijk,C., Nieuwenhuis,M.G., Posthuma,G., Thijssen,J.H., Vroom,T.M., Ahren,B., Lips,C.J., 1999. Extensive islet amyloid formation is induced by development of Type II diabetes mellitus and contributes to its progression: pathogenesis of diabetes in a mouse model. Diabetologia, 42, 427-434.

Horbach,D.A., van Oort,E., Donders,R.C., Derksen,R.H., de Groot,P.G., 1996. Lupus anticoagulant is the strongest risk factor for both venous and arterial thrombosis in patients with systemic lupus erythematosus. Comparison between different assays for the detection of antiphospholipid antibodies. Thromb. Haemost., 76, 916-924.

Horbach,D.A., van Oort,E., Tempelman,M.J., Derksen,R.H., de Groot,P.G., 1998. The prevalence of a non-phospholipid-binding form of beta2-glycoprotein I in human plasma--consequences for the development of anti-beta2-glycoprotein I antibodies. Thromb. Haemost., 80, 791-797.

Isobe,T., 1993. AA amyloidosis and AL amyloidosis. Intern. Med., 32, 919-920.

Jaroniec,C.P., MacPhee,C.E., Astrof,N.S., Dobson,C.M., Griffin,R.G., 2002. Molecular conformation of a peptide fragment of transthyretin in an amyloid fibril. Proc. Natl. Acad. Sci. U. S. A, 99, 16748-16753.

Johannessen,M., Diness,V., Pingel,K., Petersen,L.C., Rao,D., Lioubin,P., O'Hara,P., Mulvihill,E., 1990. Fibrin affinity and clearance of t-PA deletion and substitution analogues. Thromb. Haemost., %19;63, 54-59.

Kranenburg,O., Bouma,B., Kroon-Batenburg,L.M., Reijerkerk,A., Wu,Y.P., Voest,E.E., Gebbink,M.F., 2002. Tissue-type plasminogen activator is a multiligand cross-beta structure receptor. Curr. Biol., 12, 1833-1839.

Kranenburg,O., Kroon-Batenburg,L.M., Reijerkerk,A., Wu,Y.P., Voest,E.E., Gebbink,M.F., 2003. Recombinant endostatin forms amyloid fibrils that bind and are cytotoxic to murine neuroblastoma cells in vitro. FEBS Lett., 539, 149-155.

Kurusu,A., Yamada,T., Yamaji,K., Nishitani,M., Tashiro,K., Maeda,K., Horikoshi,S., Shirato,I., Rinno,H., Tomino,Y., 2004. A case of primary immunoglobulin light chain amyloidosis with a delayed appearance of Bence Jones protein in urine. Nephrology. (Carlton.), 9, 122-125.

Matsuura,E., Igarashi,Y., Yasuda,T., Triplett,D.A., Koike,T., 1994. Anticardiolipin antibodies recognize beta 2-glycoprotein I structure altered by interacting with an oxygen modified solid phase surface. J. Exp. Med., 179, 457-462.

Matzinger,P., 2002a. An innate sense of danger. Ann. N. Y. Acad. Sci., 961:341-2., 341-342.

Matzinger,P., 2002b. The danger model: a renewed sense of self. Science, 296, 301-305.

Ohgami,N., Nagai,R., Ikemoto,M., Arai,H., Kuniyasu,A., Horiuchi,S., Nakayama,H., 2001. CD36, a member of class B scavenger receptor family, is a receptor for advanced glycation end products. Ann. N. Y. Acad. Sci., 947:350-5., 350-355.

Pepys,M.B., 2001. Pathogenesis, diagnosis and treatment of systemic amyloidosis. Philos. Trans. R. Soc. Lond B Biol. Sci., 356, 203-210.

Redegeld,F.A., Nijkamp,F.P., 2003. Immunoglobulin free light chains and mast cells: pivotal role in T-cell-mediated immune reactions? Trends Immunol., 24, 181-185.

Redegeld,F.A., van der Heijden,M.W., Kool,M., Heijdra,B.M., Garssen,J., Kraneveld,A.D., Van Loveren,H., Roholl,P., Saito,T., Verbeek,J.S., Claassens,J., Koster,A.S., Nijkamp,F.P., 2002. Immunoglobulin-free light chains elicit immediate hypersensitivity-like responses. Nat. Med., 8, 694-701.

Reijerkerk,A., Mosnier,L.O., Kranenburg,O., Bouma,B.N., Carmeliet,P., Drixler,T., Meijers,J.C., Voest,E.E., Gebbink,M.F., 2003. Amyloid endostatin induces endothelial cell detachment by stimulation of the plasminogen activation system. Mol. Cancer Res., 1, 561-568.

Rostagno,A.A., Schwarzbauer,J.E., Gold,L.I., 1999. Comparison of the fibrin-binding activities in the N- and C-termini of fibronectin. Biochem. J., 338, 375-386.

Schielen,J.G., Adams,H.P., Voskuilen,M., Tesser,G.J., Nieuwenhuizen,W., 1991. Structural requirements of position A alpha-157 in fibrinogen for the fibrin-induced rate enhancement of the activation of plasminogen by tissue-type plasminogen activator. Biochem. J., 276, 655-659.

Solomon,A., 1986. Light chains of immunoglobulins: structural-genetic correlates. Blood, 68, 603-610.

Subang,R., Levine,J.S., Janoff,A.S., Davidson,S.M., Taraschi,T.F., Koike,T., Minchey,S.R., Whiteside,M., Tannenbaum,M., Rauch,J., 2000. Phospholipidbound beta 2-glycoprotein I induces the production of anti-phospholipid antibodies. J. Autoimmun., 15, 21-32.

Ursini,F., Davies,K.J., Maiorino,M., Parasassi,T., Sevanian,A., 2002. Atherosclerosis: another protein misfolding disease? Trends Mol. Med., 8, 370-374.

van Zonneveld,A.J., Veerman,H., Pannekoek,H., 1986. On the interaction of the finger and the kringle-2 domain of tissue-type plasminogen activator with fibrin. Inhibition of kringle-2 binding to fibrin by epsilon-amino caproic acid. J. Biol. Chem., 261, 14214-14218.

Yamada,M., Kadoya,Y., Kasai,S., Kato,K., Mochizuki,M., Nishi,N., Watanabe,N., Kleinman,H.K., Yamada,Y., Nomizu,M., 2002. Ile-Lys-Val-Ala-Val (IKVAV)-containing laminin alpha1 chain peptides form amyloid-like fibrils. FEBS Lett., 530, 48-52.

## Claims

1. Use of a protease inhibitor in the preparation of a medicament for the treatment of microbial infections.

2. Use of a compound binding to a cross-β structure in the preparation of a medicament for the treatment of microbial infections.

3. Use according to claim 2, wherein said compound comprises an antibody, or a functional fragment or derivative thereof, and or a finger domain of a protease, specific for a cross-β structure.

4. Use according to claim 3, wherein said antibody is a bi-specific antibody against a cross- β structure and a microbial antigen.

5. Use according to any of claims 1 to 4, wherein said microorganism is a pathogenic microorganism

6. Use according to any of claims 1 to 4, wherein a cross-β structure comprises a hydrophobin or a chaplin.

7. Use according to any of claims 1 to 6, wherein said microorganism is a fungus.

8. Use according to any of claims 1 to 6, wherein said microorganism is a gram-positive bacterium.

9. Use according to claim 8 wherein said gram-positive bacterium is an actinomycete or a streptomycete bacterium.

10. Use according to any of claims 1 to 6, wherein a cross-β structure comprises a curli or a Tafi or a thin aggregative fimbria.

11. Use according to any of claims 1 to 5 or 10, wherein said microorganism is a gram-negative bacterium.

12. Use according to claim 11 wherein said gram-negative bacterium is an *E.coli* bacterium or a *Salmorcella* bacterium.

13. A method for producing a less virulent microorganism, comprising deleting at least a part of a gene of said microorganism encoding an amyloid fibril forming protein.

14. An immunogenic composition comprising a microorganism from which at least one gene, encoding a cross-β structure forming protein, has been deleted.

15. A composition comprising a protease inhibitor and a fungicide.

16. A composition comprising a protease inhibitor and a bactericide.

17. A composition according to claims 15 or 16, wherein said protease inhibitor is a serine protease inhibitor.

18. A composition comprising a protease inhibitor and a cross-β structure-binding compound.

19. A composition according to claim 18, wherein said cross-β structure-binding compound comprises an antibody, or a functional fragment or derivative thereof, and or a finger domain of a protease, specific for a cross-β structure.

20. A composition according to claim 19, further comprising a bactericide or a fungicide.

21. A kit for detecting microbial contamination of a solution and/or a substance, said kit comprising a cross-β structure binding compound and a means for detecting binding of said cross-β structure to said binding compound.

22. A kit according to claim 21, wherein said means for detecting binding of a cross-β structure to a cross-β structure binding compound comprises a tPA- and/or factor XII activation assay.

23. A kit according to claim 21, wherein said means for detecting binding of a cross-β structure to a cross-β structure binding compound comprises visualization of a staining compound.
